Europäisches Patentamt

**(19)** European Patent Office

Office européen des brevets

**(11)** Publication number : **0 446 975 B1**

**(12)** # EUROPEAN PATENT SPECIFICATION

**(45)** Date of publication of patent specification :
02.11.94 Bulletin 94/44

**(51)** Int. Cl.⁵ : **G01N 33/24,** G01N 21/76

**(21)** Application number : **91200250.8**

**(22)** Date of filing : **06.02.91**

**(54)** Installation for the rapid analysis of tar components and method for such an analysis.

**(30)** Priority : **15.02.90 NL 9000364**

**(43)** Date of publication of application :
**18.09.91 Bulletin 91/38**

**(45)** Publication of the grant of the patent :
**02.11.94 Bulletin 94/44**

**(84)** Designated Contracting States :
**AT BE CH DE DK ES FR GB GR IT LI LU NL SE**

**(56)** References cited :
**EP-A- 0 344 950
DE-A- 3 533 173
US-A- 3 254 959
US-A- 4 540 548
JOURNAL OF THE AMERICAN CHEMICAL
SOCIETY, vol. 89, 06 December 1967; M.M.
RAUHUT et al., pp. 6515-6522**

**(56)** References cited :
**R.G.J. MILLER, "Laboratory Methods in Infrared Spectrometry", 1972, Heyden, London
(GB); R. WHYMAN, pp. 149-164
APPLIED SPECTROSCOPY, vol. 44, no. 2, February 1990; S.A. TUCKER et al., pp. 269-273**

**(73)** Proprietor : **TAUW MILIEU B.V.
Handelskade 11
NL-7417 DE Deventer (NL)**

**(72)** Inventor : **Coffa, Stuart
Brugstraat 44
NL-7607 XG Alme (NL)**
Inventor : **Urlings, Leonardus Gerardus
Catherina Matheus
Reigerlaan 15
NL-3871 TB Hoevelaken (NL)**

**(74)** Representative : **de Bruijn, Leendert C. et al
Nederlandsch Octrooibureau
Scheveningseweg 82
P.O. Box 29720
NL-2502 LS Den Haag (NL)**

EP 0 446 975 B1

## Description

The invention relates to an installation for the rapid analysis of tar components and to a method for the rapid analysis of tar components in soil.

In the case where there is a suspicion of soil contamination it is desirable rapidly to gain insight into the presence of polycyclic aromatic hydrocarbons (PAHs) (tar component) in soil, waste- and groundwater samples. Gaining rapid insight makes it possible to work more efficiently without a large number of samples having to be subjected to accurate and time-consuming testing. Depending on the result of such a soil analysis, a sample may, if desired, be subjected to a further (accurate) analysis.

The invention relates to a method for the rapid analysis of tar components in soil, wherein the soil is extracted with dichloromethane or ethyl acetate/ethanol, the extract is dried using a desiccant, and the dried extract is combined with a mixture of oxalyl chloride and dichloromethane and/or ethyl acetate and is then measured with the aid of a chemiluminescence detector and compared with a standard.

The invention also relates to an installation for carrying out this method for the rapid analysis of tar components, wherein a vessel, having a feed, a rudder motor, a window and a chemi-luminescence detector connected to a meter, the feed being connected to a vessel in which oxalyl chloride in dichloromethane and/or ethyl acetate is present.

A suitable composition of the mixture of oxalyl chloride in dichloromethane is 3 to 6% by vol. oxalyl chloride in dichloromethane and/or ethyl acetate.

It is to be recommended to add to the solution of oxalyl chloride in dichloromethane and/or ethyl acetate a solution of 2 to 4% hydrogen peroxide and $10^{-2}$ M NaOH in acetone. Of course, these two solutions are stored separately to prolong the shelf life. When extracting a soil sample with dichloromethane or ethyl acetate/ethanol and combining with a mixture of oxalyl chloride and dichloromethane and a solution of 2 to 4% hydrogen peroxide and $10^- 2$ M NaOH in acetone a flash of light is obtained. This flash of light can be measured with the aid of a chemiluminescence detector. Because of the extremely high sensitivity, in practice fluorometry is found to be less suitable than UV and chemiluminescence detection. The latter two methods are complementary. In the presence of mineral oils it is better to use UV detection, while in the absence thereof chemiluminescence detection yields better results.

The systems are calibrated with respect to an extract from a soil sample from the relevant location, which has been accurately analysed (for example high pressure liquid chromatography followed by a UV/FL detection).

By comparison with the content obtained via HPLC using UV and FL detection a calibration factor can be calculated. This factor is usually specific for a specific location.

Polycyclic aromatic hydrocarbons (PAHs) are substances which are formed on destructive distillation of coal, on fractional distillation of crude mineral oil and on incomplete combustion of these raw materials [1] M. Sittig, Handbook of Toxic and Hazardous Materials, Noyes Publications, Park Ridge, New Jersey, USA, 1981, ISBN 0-8155-08410-7.

The formulae of 16 PAHs, given in the EPA (Environmental Protection Agency) list, are given in the following table.

## Table A

The detection limits maintained, which are given in the following table, are expressed in µg/l for water and in mg/kg of solids for soil.

| Component | µg/l | mg/kg of solids |
|---|---|---|
| 1. Naphthalene | 0.05 | 0.05 |
| 2. Acenaphthalene | 0.05 | 0.05 |
| 3. Acenaphthene | 0.05 | 0.05 |
| 4. Fluorene | 0.05 | 0.05 |
| 5. Phenanthrene | 0.01 | 0.01 |
| 6. Anthracene | 0,01 | 0.01 |
| 7. Fluoroanthene | 0.01 | 0.01 |
| 8. Pyrene | 0.01 | 0.01 |
| 9. Benz(a)anthracene | 0.01 | 0.01 |
| 10. Chrysene | 0.01 | 0.01 |
| 11. Benzo(b)fluoroanthene | 0.01 | 0.01 |
| 12. Benzo(k)fluoroanthene | 0.01 | 0.01 |
| 13. Benzo(a)pyrene | 0.01 | 0.01 |
| 14. Benzo(ghi)perylene | 0.01 | 0.01 |
| 15. Dibenz(a,h)anthracene | 0.01 | 0.01 |
| 16. Indeno(1,2,3-cd)pyrene | 0.01 | 0.01 |

Because of the carcinogenic properties, these PAHs are a health hazard for man. It is therefore of the greatest importance that in the event of a contamination situation the area contaminated with PAHs can be marked off relatively rapidly. For this purpose a good analytical chemical laboratory must have a relatively rapid field analysis method at its disposal.

The requirements which a method of this type must meet are:

1. selective with respect to PAHs,
2. quantitative in the sense that all 16 PAHs are determined,
3. relatively accurate,
4. sufficiently sensitive to be able to differentiate between B and C value (see Table B, page 22)
5. reproducible,
6. relatively simple and fast,
7. easily transportable system,
8. relatively inexpensive
   (see Table B).

Important points for consideration during the development of a field analysis method, which is suitable in principle, for the determination of the total PAHs content are:

1. heterogeneity of the soil sample,
2. extraction efficiency of the extractant,
3. extraction time,
4. composition of the PAHs present in connection with the individual sensitivity/intensity (absorption or emission) of the PAHs,
5. interference due to the presence of mineral oil or other components which to a limited extent dissolve in the extractant (distribution of the PAHs between the various phases).

The above implies that, in fact, different analytical methods can be compared with one another only if and then only well if the same extract is always used as the starting material. Studies showed that this is a necessary but not sufficient precondition.

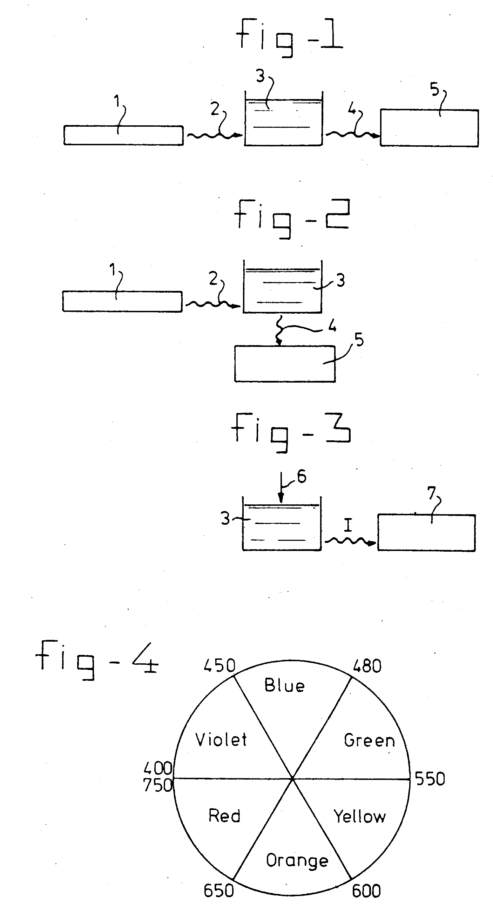

The current rapid test for PAHs is based on the fact that a contamination by PAHs is frequently associated with the presence of coloured co-contaminants (degradation products of PAHs?) and/or anthracene. The majority of PAHs are completely transparent and can thus not simply be detected visually. Following an evaluation of the rapid test, it was found that this test is insufficiently selective, gives fluctuating results and tends to overestimate the total content of PAHs. (Own evaluation.) Changing from visual subjective detection to objective detection by instrument gives an improvement. Three determination techniques using instruments were tested to assess their applicability. These techniques are: ultraviolet spectrometry (UV), fluorescence spectrometry (FL) and chemiluminescence detection (CL). They are shown diagrammatically in Figures 1-3.

Figure 1 shows the use of ultraviolet spectrometry. In this figure 1 is a UV light source, 2 is the incident light, 3 indicates the cell, 4 is the emergent light and 5 the detector.

Figure 2 shows fluorescence spectrometry, in which 1 is again the light source, which is very intense, 2 is the light $I_o$ incident on the cell, 3 is the cell, 4 is the fluorescence light and 5 is the detector. It is an emission measurement which is concerned here, while Figure 1 shows an absorption measurement.

Figure 3 shows chemiluminescence detection, in which a reagent 6 is added to a cell 3, which results in a light emission (I) which is measured by a detector 7. In this case therefore the measurement is an emission measurement without an external light source. The relationship between absorption and colour is shown in Figure 4. If a compound absorbs violet radiation (400-500 nm), the compound is yellow (550-600 nm) in colour. The converse is also true. Violet and yellow are then said to be complementary colours.

Compounds which do not absorb any radiation between 400 and 800 nm are colourless or white.

To the left of the visible region, a differentiation is made between the far UV (<200 nm) and the near UV ( 200-400 nm). In the text which follows only the near UV is illustrated because the spectrometer used emits UV light with a wavelength of 254 nm. All compounds which absorb at this wavelength are determined. No differentiation is made between, for example, benzene (and derivatives) and PAHs. UV is thus not very selective. However, it is a suitable method for quantitative analysis. If a bundle of monochromatic light (light with one wavelength) is allowed to fall through a medium in which molecules of a substance are present which absorb this light (Figure 1), then:

$$I = I_o \exp - E.d.c. \quad (1)$$

In this equation:

$I_o$    = intensity of incident light,
$I$     = intensity of emergent light,
$d$    = optical path length (cm)
$E$    = molecular extinction coefficient ($m^3$/mol.cm)
$c$    = concentration of the absorbing substance (mol/$m^3$)

In Figure 5 relative intensity ($I/I_o$) after absorption is shown as a function of E.d.c.

In Figure 6 fluorescence radiation after excitation is shown.

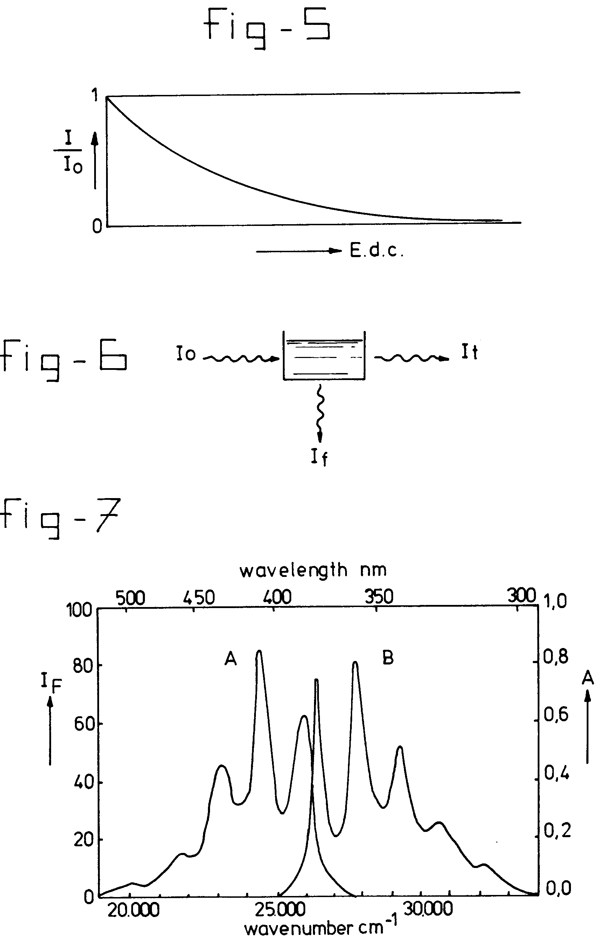

By absorption of a radiation photon, a molecule will be brought to a higher energy level (excitation). On returning to a lower energy level, the difference in energy is converted to radiation. In general, this fluorescence radiation does not have to have the same wavelength as the absorbed radiation. A complete fluorescence spectrum is emitted which is shifted to a smaller frequency (longer wavelength). In this context see Figure 7, in which the fluorescence spectrum (A) and the absorption spectrum (B) of anthracene, [3] L. de Galan, Analytische Spektrometrie (Analytical Spectrometry), Agon Elsevier Amsterdam, ISBN 901010224, 1972, are shown.

The intensity of the emergent light in the longitudinal direction of the incident light is, according to (1)

$$I_t = I_o \exp - E\,d\,c \quad (4)$$

The intensity of the fluorescence radiation is then

$$I_f = f\,(I_o - I_t) = f\,I_o\,(1 - \exp - E\,d\,c) \quad (5)$$

In this equation f represents the fluorescence yield. In favourable cases $0.1 \leqq f \leqq 0.6$.

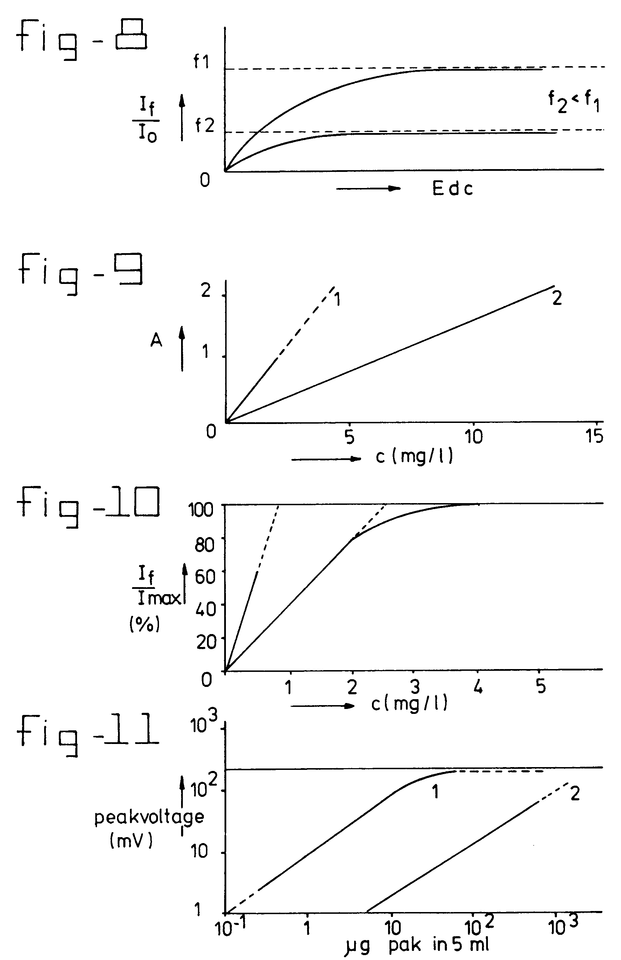

In Figure 8 the relative fluorescence intensity is shown as a function of E d c and of the fluorescence yield.

$$\text{If } E\,d\,c \ll 1 \text{ or } c \ll {}^1\!/E\,d, \text{ then } I_f = f\,I_o\,E\,d\,c \quad (6).$$

In order to gain an impression with regard to the maximum permissible concentration of each PAH, because (6) applies it is postulated that

$$C_{max} = 0.1 / E\,d \quad (7)$$

If d = 1 cm and the solvent is acetonitrile, then, according to [7], M. Vos, Indikatieve veldtest op olie en PAK's (Indicative field test for oil and PAHs), work experience report in environmental laboratory TAUW, June 1988, the following table applies.

## Table C

Maximum permissible PAH concentration in acetonitrile ($\bullet$ = 254 nm) in the case of fluorescence spectrometry [4]

National Bureau of Standards Certificate of Analysis Standard Reference Material 1647. Priority Pollutant Polynuclear Aromatic Hydrocarbons (in acetonitrile).

| No. | Component | E ($m^3$/mol.cm) | $C_{max}$ (mg/l) |
|---|---|---|---|
| 1. | Naphthalene | 3.1 | 4 |
| 2. | Acenaphthylene | 2.2 | 8 |
| 3. | Acenaphthene | 1.2 | 12 |
| 4. | Fluorene | 17 | 1 |
| 5. | Phenanthrene | 43 | 0.4 |
| 6. | Anthracene | 96 | 0.2 |
| 7. | Fluoroanthene | 13 | 0.4 |
| 8. | Pyrene | 10 | 2 |
| 9. | Benz(a)anthracene | 33 | 0.7 |
| 10. | Chrysene | 52 | 0.4 |
| 11. | Benzo(b)fluoroanthene | 43 | 0.5 |
| 12. | Benzo(k)fluoroanthene | 28 | 1 |
| 13. | Benzo(a)pyrene | 42 | 0.6 |
| 14. | Benzo(g,h,i)perylene | 16 | 2 |
| 15. | Dibenz(a,h)anthracene | 11 | 2 |
| 16. | Indeno(1,2,3-cd)pyrene | 38 | 0.7 |

It is thus found that anthracene, chrysene, phenanthrene, benzo(b)fluoroanthene and fluoroanthene make the largest contribution to the fluorescence intensity. The contribution of naphthalene, acenaphthylene and acenaphthene is negligible. It is to be expected that the above statements will remain valid if dichloromethane is taken as the solvent in place of acetonitrile.

In certain cases "quenching" can occur if the total PAH concentration is relatively high. This term is understood to mean that molecules which have arrived in an excited state lose their energy by interaction with other PAHs which are in the base state, as a result of which the fluorescence yield is reduced [2] W.J. Moore, Physical Chemistry, 5th edition, Longman, 1974. Diluting until a proportional fall in the fluorescence intensity occurs is then the only means of establishing whether measurements are being carried out in the straight line region. In practice this will definitely create problems, certainly in the case of large numbers of soil samples.

## Chemiluminescence detection

Stated very simply, chemiluminescence is a chemical reaction in which light is emitted. Schematically, this can be represented as follows

$$(1) \quad OC + P \longrightarrow > I$$
$$(2) \quad Flu + I \longrightarrow > Flu^* \text{excitation}$$
$$(3) \quad Flu^* \longrightarrow > Flu + \text{lightemission}$$

In the first reaction an "energy-rich" intermediate is formed. his intermediate then gives up energy to a fluorophore, which as a result is brought into an excited state (reaction 2). On return to the base state (reaction 3) light is emitted.

Reaction 2 is reasonably selective because only a restricted number of substances can be excited. These include polycyclic aromatic hydrocarbons (PAHs), amino-PAHs, corticosteroids, labelled carboxylic acids, labelled alkylamines, dansylated amino acids and fluorescamine-labelled catecholamines [8]. C. Semeins, Chemiluminescentie als detektiemethode in vloeistofchromatografie, een literatuurscriptie (Chemiluminescence as a detection method in liquid chromatography: a literature review), University of Amsterdam, 1986.

The above reaction scheme can now be made more specific.

$$\text{base}$$
$$(1) \quad (COCl)_2 + H_2O_2 \quad \blacksquare\!\!-\!\!-\!\!\blacksquare> \quad C_2O_4$$
$$(2) \quad PAH + C_2O_4 \quad \blacksquare\!\!-\!\!-\!\!-\!\!\blacksquare> \quad PAH^* \ (+2 \ CO_2) \ \text{excitation}$$
$$(3) \quad PAH^* \quad \blacksquare\!\!-\!\!-\!\!-\!\!-\!\!\blacksquare> \quad PAH + \text{light} \qquad \text{emission}$$

Because of the very limited shelf life (<24 hours) of a mixture of ($COCl_2$) and $H_2O_2$ (oxalyl chloride and hydrogen peroxide respectively), it is sensible to adapt the above reaction scheme a little, as follows.

$$(COCl)_2$$
$$(I) \quad PAH + H_2O_2 + base \quad \blacksquare\!\!-\!\!-\!\!-\!\!\blacksquare> \quad PAH^* \ (+2 \ CO_2) \ \text{excitation}$$
$$(II) \quad PAH^* \quad \blacksquare\!\!-\!\!-\!\!-\!\!\blacksquare> \quad PAH + \text{light} \qquad \text{emission}$$

By storing the reagents separately, the shelf life is (appreciably) prolonged. Thus, oxalyl chloride in dichloromethane (4% by vol.) is definitely stable for 3-4 weeks. A limit of 1 week applies for hydrogen peroxide/NaOH (3% by weight and, respectively, 0.01 M in acetone). The two solutions must be stored cool (4°C) and in the dark.

## Method

## Calibration of the measurement system

It is in fact very difficult to calibrate a measurement system for a mixture of PAHs. After all, the individual contribution of each PAH differs from sample to sample [4,5]. National Bureau of Standards Certificate of Analysis Standard reference material 1647. Priority Pollutant Polynuclear Aromatic Hydrocarbons (in acetonitrile), E.A. Hogendoorn, Analyse van PAK's in milieumonsters: enkele ontwikkelingen (Analysis of PAHs in environmental samples: a few developments). Laboratorium Praktijk, June/July 1988, 328-330. Nevertheless, as a compromise the correction factor for the relevant location is determined on the basis of the analysis of several samples by the environmental laboratory.

## The following test method was used

1. Calibration on a limited number of PAHs, specifically Nos. 1 to 7 inclusive and 9
2. Calibration on an extract from one soil sample (VOORST 5). The total PAH concentration was determined via HPLC using UV/FL detection;
3. Correction for the location examined (Example I).

## Preparation of the extracts

1. Mix a soil sample well.
2. Weigh 20 g into a container (jam jar) (370 ml capacity). Repeat this in triplicate to check for inhomogeneity [6] J.C. Miller, J.N. Miller "Basic Statistical Methods for Analytical Chemistry, Part 1. Statistics of repeated measurements. A review. Analyst, September 1988, vol. 113, 1351-1355."
3. Add 100 ml of dichloromethane or ethyl acetate/ethanol (4:1 v/v) and then 10 g of $MgSO_4$ to each portion.
4. Shake for about 2 minutes.
5. Filter through fluted filter paper.
6. Collect the filtrates (extracts) in glass containers with a capacity of about 100 ml (minimal "head space").
7. Store the extracts in the dark and cool.

## Measurement of calibration solutions and soil sample extracts

### 1. UV spectrometry (1 cm quartz cell)

1. Record an absorption spectrum by measuring the extinction of a PAH mixture in dichloromethane (7.4 mg of PAH/l) or ethyl acetate/ethanol (4:1 v/v) as a function of the (excitation) wavelength. Determine the absolute absorption maximum.
2. Determine the range of the calibration line by measuring extinctions of a number of solutions (0.38/0.76/1.9/3.8/7.4/11.4/19 and 38 mg PAH/litre) at that wavelength at which maximum absorption occurred (see 1).
3. Measure the extinctions of the extracts obtained (for example 45 Zeist, 1 Voorst 5), after dilution if necessary.

### 2. Fluorescence spectrometry

1. Adjust the excitation wavelength to 254 nm and the emission wavelength to 390 nm.
2. Set the fluorimeter to 100% emission using a solution which contains a maximum of 19 mg of PAH/l (see Table 1). Set to 0% using dichloromethane or ethyl acetate/ethanol (4:1 v/v).
3. Measure the emissions of the other calibration solutions (0.38/0.76/1.9/3.8/7.4 and 11.4 mg PAH/l).
4. If the relationship between emission and concentration is not linear, step 2 must be repeated using a solution which contains 11.4 mg of PAH/l.
   Then measure the emissions of the other calibration solutions (0.38-7.4 mg of PAH/l). This must be repeated until a proportional fall in the emission with the concentration is detected.
5. Measure the emissions of the extracts obtained, after dilution if necessary.

## Chemiluminescence detection

1. Transfer 4 ml of oxalyl chloride into a 100 ml volumetric flask and make up with dichloromethane or ethyl acetate (solution A).
2. Transfer 10 ml of 30% by weight hydrogen peroxide and 1 ml of 1 molar sodium hydroxide into a 100 ml volumetric flask and make up with acetone.
   Then filter through a fluted filter (solution B).
3. Pipette 5 ml of a calibration solution, which contains 0.38 mg of PAH/l, and 1 ml of solution B into a cell which has a capacity of about 10 ml. Place a magnet stirrer in the cell.

4. Place the cell in front of a photocell and over a magnetic stirrer. Start stirring.

5. Close the measurement unit light-tight.

6. Start the recorder (measurement range 10 mV).

7. Inject 1 ml of solution A into the cell using an ejector system.

8. Determine the maximum photovoltage.

9. Repeat operations 3 to 8 inclusive with the other calibration solutions (0.76/1.9/3.8/7.4/11.4/19 and 38 mg of PAH/l).

The measurement range of the recorder must now be adjusted to 50 or 100 mV.

Now calibrate on an extract, from a soil sample, which contains 26 mg of PAH/l.

10. For this purpose prepare dilute solutions which contain 0.13/0.52/1.0/1.3 and 2.6 mg of PAH/l.

11. Pipette 5 ml of the first solution B into the cell.

12. Place the cell (+ magnetic stirrer) in front of the photocell and over the magnetic stirrer. Start stirring.

13. Close the measurement unit light-tight.

14. Start the recorder (measurement range 10 mV).

15. Inject 1 ml of solution A into the cell.

16. Determine the maximum photovoltage.

17. Repeat operations 11 to 15 inclusive with the other solutions, the measurement range of the recorder being adjusted to 50, 100 or 500 mV.

18. Measure the emissions of the extracts obtained, after dilution if necessary.

The results obtained from the UV measurements are given in Figure 9, in which the extinction is shown as a function of the PAH concentration at 254 nm.

## Table D

Concentration of PAHs in soil samples, originating from 3 locations, determined by means of UV spectrometry compared with HPLC/UV, FL results

| Method | [PAH] | (mg/kg of solids) | HPLC |
|---|---|---|---|
| | calibration line 1 | calibration line 2 | |
| Detection | UV | UV | UV, FL |
| Sample No. | | | |
| 84 (Vos 2) | 290±50 | 1070±160 | 53 |
| 86 (Vos 4) | 200±20 | 750±70 | 27 |
| 90 | 370±90 | 1350±320 | 92 |
| 93 | 300±70 | 1110±250 | 120 |
| 94 | 1080±70 | 4020±250 | 270 |
| 95 | 430±80 | 1610±290 | 200 |
| 96 | 610±80 | 2250±290 | 94 |
| 111 | 150±20 | 550±70 | 69 |
| 114 | 510 | 1890 | 71 |
| | 250 | 930 | 71 |
| | 260 | 950 | |
| 116 | 420 | 1560 | 290 |
| | 310 | 1170 | |
| | 1550 | 5760 | |
| 3516.1 | 82±3 | 310±10 | 3.1 |
| 3516.2 | 66±7 | 250±30 | 14 |
| 3847 | 390±10 | 1450±40 | 0.4 |
| 4113 | 2010±570 | 7500±2100 | 3300 |

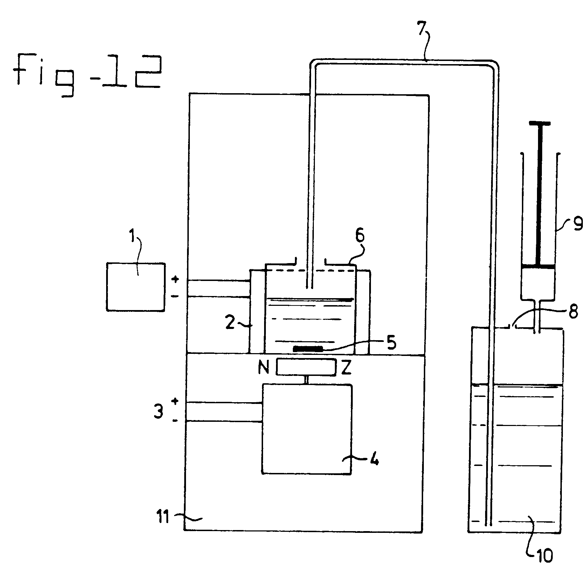

In Figure 10 the relative fluorescence intensity is shown as a function of the PAH concentration ($\lambda_e$xc = 254 nm, $\bullet_e$m = 390 nm). In Fig. 11, 1 shows the calibration line for extract obtained from VOORST 5 and 2 the calibration line for a solution of PAH 1 to 7 inclusive and 9 (EPA).

In view of the above result, it would be possible to increase the range of calibration line 2 by setting the fluorimeter to 100% using the calibration solution which contains 1.9 mg of PAH/l. This signifies that only soil sample extracts having a content of PAHs of less than 10 mg/kg of solids can be measured undiluted. For calibration line 1 the limit is at 2 mg/kg, i.e. about the A value.

This detection method is thus in fact too sensitive to investigate whether the PAH content is at the C level (200 mg/kg of solids). Moreover, in the presence of a relatively large amount of mineral oil the risk of (gross) overestimation of the PAH content is very high [7] M. Vos, "Indikatieve veldtest op olie en PAK's. Verslag stage binnen milieulab. TAUW, juni 1988" ("Indicative field test for oil and PAHs. Report on work experience in environmental laboratory, TAUW, June 1988"). See also 3.4.2.

For these reasons, the fluorimeter is less suitable as field analysis equipment for determining the PAH content.

In Figure 11 the maximum photovoltage during chemiluminescence is shown as a function of the amount of PAH in 5 ml of dichloromethane. In this case 1 is the extract from Voorst 5 and 2 is the calibration solution containing PAH 1 to 7 inclusive and 9.

The saturation voltage of the photocell used is about 250 mV. However, the straight-line relationship between maximum photovoltage and PAH concentration is already lost at about 150 mV. For this reason, the amount of sample processed is such that the maximum photovoltage is between 1 and 100 mV.

For calibration line 1, 1 mV corresponds to $81 \pm 6$ ng of PAH.

For calibration line 2, the corresponding figure is $7.2 \pm 0.7$ µg of PAH/mV.

Table E

Content of PAHs in soil samples, originating from 3 locations, determined by means of chemiluminescence detection compared with HPLC/UV, FL results

| Method | [PAH] calibration line 1 | (mg/kg of solids) calibration line 2 | HPLC |
|---|---|---|---|
| Detection | CL | CL | UV, FL |
| Sample No. | | | |
| 84 (Vos 2) | 9±1 | 780±120 | 53 |
| 86 (Vos 4) | 7±1 | 590±80 | 27 |
| 90 | 26±4 | 2370±340 | 92 |
| 93 | 13±5 | 1130±410 | 120 |
| 94 | 155±15 | 13900±1370 | 270 |
| 95 | 42±6 | 3770±550 | 200 |
| 96 | 64±3 | 5790±240 | 94 |
| 111 | 7±2 | 600±170 | 69 |
| 114 | 66±12 | 5790±1110 | 71 |
| | 8±3 | 690±280 | |
| | 12±3 | 1110±280 | |
| 116 | 69±13 | 6200±1150 | 290 |
| | 47±10 | 4180±870 | |
| | 1440±260 | 130000±23000 | |
| 3516.1 | 0.8±1 | 70±8 | 3.1 |
| 3516.2 | 0.3±0.1 | 27±7 | 14 |
| 3847 | 0.26±0.02 | 23±2 | 0.4 |
| 4113 | 5.7±0.2 | 510±20 | 3300 |

Table F

Content of PAHs in soil samples, originating from 3 locations, determined by means of UV spectrometry and chemiluminescence detection compared with HPLC/UV, FL results.

N.B.

Sample No. 4113 contains a relatively large amount of mineral oil.

| Method | (rapid analysis) (calibration line 1)/1.3 | (rapid analysis) (calibration line 1)*5 | (environmental lab) HPLC |
|---|---|---|---|
| Detection | UV | CL | UV, FL |
| Sample No. | | | |
| 84 (Vos 2) | 220±50 | 45±1 | 53 |
| 86 (Vos 4) | 150±20 | 35±1 | 27 |
| 90 | 280±90 | 130±4 | 92 |
| 93 | 230±70 | 65±5 | 120 |
| 94 | 830±70 | 775±15 | 270 |
| 95 | 330±80 | 210±6 | 200 |
| 96 | 470±80 | 320±3 | 94 |
| 111 | 120±20 | 35±2 | 69 |
| 114 | 390 | 330±12 | 71 |
| | 190 | 40±3 | |
| | 200 | 60±3 | |
| 116 | 320 | 345±13 | 290 |
| | 240 | 235±10 | |
| | 1190 | 7200±260 | |
| 3516.1 | 63±3 | 4.0±0.1 | 3.1 |
| 3516.2 | 51±7 | 1.5±0.1 | 14 |
| 3847 | 300±10 | 1.30±0.02 | 0.4 |
| 4113 | 1550±570 | 28.5±0.2 | 3300 |

Table G

Reliability of UV and chemiluminescence detection compared with HPLC followed by UV fluorescence detection.

Conclusions

|  | Correct (%) | Over-estimate (%) | Under-estimate (%) |
|---|---|---|---|
| UV | 50-70 | 23-50 | 0-7 |
| CL | 90 | 0-10 | 0-10 |
| Visual | 50 | 35 | 15 |

It follows from the above that:
- Correction for the location is necessary.
- Compared with CL detection, UV detection gives an overestimate of the PAH content in soil samples in more cases.
- In the case of relatively high contents of mineral oil in soil samples, UV detection will be preferable to CL detection.
- UV detection and CL detection are complementary.
- UV detection and CL detection are good methods for the determination of relative PAH concentrations in samples.
- Screening of samples for PAHs can be carried out more easily by the use of CL or UV detection.
- 2 of the 14 soil samples were inhomogeneous and even remained inhomogeneous after intensive mixing.
- CL detection gives a better result than visual observations.

A suitable method is as follows:

Indicative shaking test supplemented bv chemiluminescence detection

Reagents:

A. 4% by volume oxalyl chloride in $CH_2Cl_2$ (DCM) or ethyl acetate
B. 3% by weight $H_2O_2/10^{-2}$ M NaOH in acetone

Soil samples:

20 g in 100 ml of DCM or ethyl acetate/ethanol (4:1 v/v) with 10-20 g of $MgSO_4$ as desiccant. After shaking for 2 minutes filter through a fluted filter.

In a cell having a capacity of about 10 ml:

inject
- 100 $\mu$l of extract (using injection syringe)
- 5.0 ml of DCM or ethyl acetate (using dispenser)
- 1 ml of solution B (using Eppendorf pipette or dispenser)
- while stirring, 1 ml of solution A.

Measure the maximum photovoltage of each extract (measurement range of recorder 100 mV). Submit 1 extract to the environmental laboratory to enable the calibration factor to be calculated ($\mu$g of PAH/mV). Calculate the other PAH concentrations in the soil samples (number of mV * calibration? factor * 50 ,— , >mg of PAH/kg).

In the examples which follow an installation for carrying out the rapid analysis according to the invention is illustrated. A measuring instrument for carrying out the PAH/field analysis expediently consists of three units, i.e.:
1. the chemical unit with sensor and mixing motor
2. display unit
3. battery unit

The display unit records and indicates the signal which originates from the sensor, which is located in the box in which the chemical reaction is carried out. The lead-through for the power supply for the motor is also located in this box. The power supply for the entire instrument and the on/off switch for the entire instrument are in the battery box.

Functions:

- "hold" function, by which means the measured value remains unchanged, by which means ample time is available for recording the measured value
- an indicator for a "low" battery
- a low energy consumption is achieved by the use of a LCD display
- environmentally friendly as a result of the use of a rechargeable battery
- battery charge connection, as a result of which the battery does not have to be removed in order to be charged.

It was found that in practice a unit of this type can operate for more than 8 hours on a single battery charge.
During operation of the unit, the chemical unit is connected to the display unit.
The battery box is connected to the display unit and the three units are housed in a suitable case.
A measurement can be carried out in the following way:
1. Connect the instrument to the battery box
2. Check indicator for "low" battery
3. Set the ranges switch to the desired range
4. Press in the "start" button
5. Allow the chemical reaction to proceed (with stirring)
6. Record the value read off
7. If desired press the "hold" button so that the value read off remains unchanged even after any change has occurred in the chemical unit
8. Rinse the cell with (dichloromethane or ethyl acetate) and then allow to leak out on filter paper
9. Re-start at operation 4) or if desired at 3).
The installation used for the chemiluminescence system is shown in Fig. 12.
In this figure 1) is the recording apparatus (0 to 100 mV), 2) the photocell (0 to 150 mV), 3) the battery (1.5 V), 4) a DC motor, 5) a magnet (stirrer), 6) a glass cell (10 ml) containing an extract/$H_2O_2$/NaOH, 7) a Teflon capillary ($\phi$ = 1 mm), 8) a vent, 9) an injection syringe (0 to 5 ml), 10) a glass container containing oxalyl chloride in dichloromethane or ethyl acetate, and 11) a Teflon-coated metal casing to be closed by a metal plate, likewise coated with Teflon.

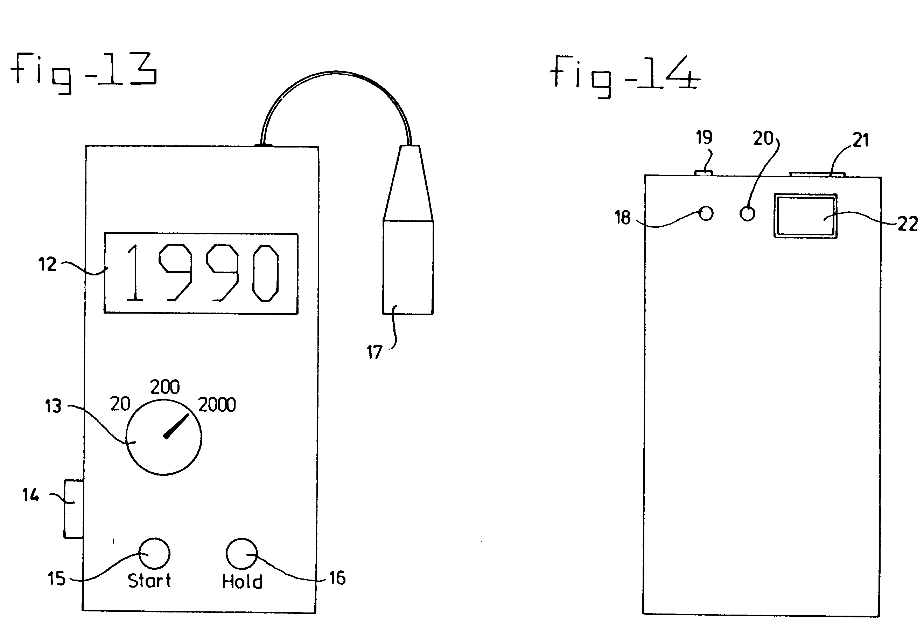

In Fig. 13 the display unit is shown. This unit is provided with a display 12, a range selector 13, a power supply for the motor as well as signal input 14, a start button 15, a "hold" button 16 and a screw connector for the power plug 17. In Fig. 14 the battery box is shown, which is provided with a red LED for indicating that the voltage of the cell 18 is too low, a socket 19, an on and off indicator with a green LED 20, a power supply for the motor 21 and an on/off switch 22.
The various features are explained with reference to the following examples.

Example I

As described above, soil samples are shaken with dichloromethane or ethyl acetate/ethanol (4:1 v/v) in order to dissolve PAHs present. It is now assumed that the relative molar ratio of the PAHs in the extract is identical to that in the original soil sample. A so-called theoretical response is then calculated for each PAH in the sample. The procedure is as follows. Calculate the molality of a PAH by dividing the percentage by weight thereof by the molecular mass. The molalities are obtained in this way. Then multiply this result by the molecular extinction coefficient of the relevant PAH at 254 nm if UV spectrometry is used (see Table 1 on page 6). This product can be regarded as a theoretical extinction in a 1 cm cell.
The total theoretical extinction is the sum of the individual contributions. By now plotting the value obtained against the PAH content determined by the environmental laboratory, a straight line is obtained (with a direction coefficient of 150 ml/mg of PAH). For sample VOORST 5 a content of 130 mg of PAH/kg was found. This corresponds to a theoretical extinction of 19.5 l/kg. If the theoretical extinction is now calculated on the basis of the PAH composition in this sample, a value of 25.4 l/kg is then found. This signifies that calibration on calibration line 1 will give too high a value. The PAH contents found when using UV spectrometry must thus be divided by 1.3 for this location.
In the case of chemiluminescence, all emission wavelengths are detected by the photocell. The theoretical response is now calculated by multiplying the molality of each PAH by the maximum extinction coefficient as indicated in [4], National Bureau of Standards Certificate of Analysis Standard Reference Material 1647. Priority Pollutant Polynuclear Aromatic Hydrocarbons (in acetonitrile). After summation, a (theoretical) response/(PAH) content plot is obtained with a straight line with a direction coefficient of 360 ml/mg of PAH. For sample VOORST 5 which has a content of 130 mg of PAH/kg, this corresponds to a theoretical response of 46.8 l/kg. On the basis of the PAH composition, a value of 9.2 l/kg is found. This signifies that calibration on calibration line 1 will give too low a value. The PAH contents found when using CL detection must thus be multiplied by 5 for this location.

Example II

PAH rapid analysis using ethyl acetate/ethanol
Aim: Determination of the suitability of ethyl acetate/ ethanol for the PAH field test
Method: Samples which have been analysed for the 16 PAH according to EPA (see Table A) are diluted with ethyl acetate/ethanol (4:1 v/v) instead of dichloromethane and are then quantified using the rapid analysis. Analysis by the HPLC method was used as a check.

| | Rapid analysis | | Shaking test | | |
|---|---|---|---|---|---|
| Results | PAH (corrected) | | (16) EPA-PAH | quantitative | |
| | mg/kg of solids | | mg/kg of solids | DCM | E/E |
| MM 1 | 138 (± 10%) | 13 | dubious | dubious | |
| MM 2 | 45 | " | 67 | coloured | coloured |
| MM 3 | 11 | " | 12 | dubious | dubious |
| MM 16 | 7 | " | 5 | dubious | dubious |
| MM 5 | 1 | " | 0 | colour-less | colour-less |
| MM 6 | 3 | " | 5 | dubious | dubious |
| MM 20 | 8 | " | 2 | dubious | dubious |
| MM 8 | 8 | " | 1 | colour-less | colour-less |

Conclusion: A good differentiation between soil contaminated with PAH and soil not contaminated with PAH can (also) be made using the extractant ethyl acetate/ ethanol E/E. The PAH rapid analysis and the EPA PAH analysis agree with one another on the whole. (EPA = Environmental Protection Agency)

## TABLE B

Check table for assessing the concentration levels of various contaminants in the soil

Indicative guidelines: A - reference value

B - check value for (further) investigation

C - check value of clean-up (investigation)

| component level | Occurrence in Soil, mg/kg of solid | | | Groundwater (µg/l) | | |
|---|---|---|---|---|---|---|
| | A* | B | C | A | B | C |
| Polycyclic hydrocarbons | | | | | | |
| 1. Naphthalene | 0.1 | 5 | 50 | 0.2 | 7 | 30 |
| 6. Anthracene | 0.1 | 10 | 100 | 0.1 | 2 | 10 |
| 5. Phenanthrene | 0.1 | 10 | 100 | 0.1 | 2 | 10 |
| 7. Fluoroanthene | 5 | 0.1 | 10 | 100 | 0.02 | 1 |
| 8. Pyrene | 0.1 | 10 | 100 | 0.02 | 1 | 5 |
| 13. Benzo(a)pyrene | 0.05 | 1 | 10 | 0.01 | 0.2 | 1 |
| PAHs (total) | 1 | 20 | 200 | 0.2 | 10 | 40 |

## Claims

1. Method for the rapid analysis of tar components in soil, **characterised in that** the soil is extracted with dichloromethane or ethyl acetate/ethanol, the extract is dried using a desiccant, and the dried extract is combined with a mixture of oxalyl chloride and dichloromethane and/or ethyl acetate and is then measured with the aid of a chemiluminescence detector and compared with a standard.

2. Method according to Claim 1, **characterised in that** 3 to 6 % by volume of oxalyl chloride in dichloromethane and/or ethyl acetate is used.

3. Method according to Claim 1 or 2, **characterised in that** a solution of 2 to 4 % by weight of hydrogen peroxide in $10^{-2}$ molar NaOH in acetone is added to the solution of oxalyl chloride in dichloromethane and/or ethyl acetate.

4. Installation for the rapid analysis of tar components, as claimed in any of claims 1-3, **characterised by** a vessel, having a feed, a rudder motor, a window and a chemiluminescence detector connected to a meter, the feed being connected to a vessel in which oxalyl chloride in dichloromethane and/or ethyl acetate is present.

**Patentansprüche**

1. Verfahren zur Schnellanalyse von Teerbestandteilen im Erdboden, dadurch gekennzeichnet, daß der Erdboden mit Dichlormethan oder Ethylacetat/-Ethanol extrahiert wird, der Extrakt unter Verwendung eines Trockenmittels getrocknet wird und daß der getrocknete Extrakt mit einer Mischung aus Oxalylchlorid und Dichlormethan und/oder Ethylacetat kombiniert und dann mit Hilfe eines Chemilumineszenzdetektors gemessen und mit einem Standard verglichen wird.

2. Verfahren nach Anspruch 1, dadurch gekennzeichnet, daß 3 bis 6 Volumen-% Oxalylchlorid in Dichlormethan und/oder Ethylacetat verwendet werden.

3. Verfahren nach Anspruch 1 oder 2, dadurch gekennzeichnet, daß eine Lösung von 2 bis 4 Gew.-% Wasserstoffperoxid in $10^{-2}$ molarer NaOH in Aceton zu der Lösung von Oxalylchlorid in Dichlormethan und/oder Ethylacetat gegeben wird.

4. Vorrichtung zur Schnellanalyse von Teerbestandteilen, nach einem der Ansprüche 1 bis 3, gekennzeichnet durch einen Behälter mit einem Einlaß, einem Rührmotor, einem Fenster und einem mit einem Meßgerät verbundenen Chemiluminiszenzdetector, wobei der Einlaß mit einem Behälter verbunden ist, in dem Oxalylchlorid in Dichlormethan und/oder Ethylacetat vorhanden ist.

**Revendications**

1. Procédé pour l'analyse rapide des composants du goudron dans la terre, caractérisé en ce qu'on extrait la terre avec du dichlorométhane ou de l'acétate d'éthyle/éthanol, on sèche l'extrait à l'aide d'un dessiccant et on combine l'extrait séché à un mélange de chlorure d'oxalyle et de dichlorométhane et/ou d'acétate d'éthyle, puis on mesure à l'aide d'un détecteur de chimioluminescence et on compare à une référence.

2. Procédé selon la revendication 1, caractérisé en ce qu'on utilise 3 à 6% par volume de chlorure d'oxalyle dans le dichlorométhane et/ou l'acétate d'éthyle.

3. Procédé selon la revendication 1 ou 2, caractérisé en ce qu'on ajoute une solution de 2 à 4% par poids de peroxyde d'hydrogène dans NaOH $10^{-2}$ molaire dans l'acétone à la solution de chlorure d'oxalyle dans le dichlorométhane et/ou l'acétate d'éthyle.

4. Installation pour l'analyse rapide des composants du goudron selon l'une quelconque des revendications 1 à 3, caractérisée en ce qu'elle comprend une cuve équipée d'un dispositif d'alimentation, d'un agitateur, d'une fenêtre et d'un détecteur de chimioluminescence raccordé à un appareil de mesure, le dispositif d'alimentation étant relié à une cuve contenant le chlorure d'oxalyle dans le dichlorométhane et/ou l'acétate d'éthyle.

fig-1

fig-2

fig-3

fig-4

# Fig - 5

# Fig - 6

# Fig - 7

fig - 8

$\frac{I_f}{I_0}$

f1

f2

$f_2 < f_1$

0

Edc

fig - 9

A

2

1

1

2

0

5

10

15

c (mg/l)

fig - 10

$\frac{I_f}{I_{max}}$ (%)

100

80

60

40

20

0

1

2

3

4

5

c (mg/l)

fig - 11

$10^3$

peakvoltage (mV)

$10^2$

1

2

10

1

$10^{-1}$

1

10

$10^2$

$10^3$

µg pak in 5 ml

fig-12

fig-13

12 — 1990

13 — 200 / 20 / 2000

14

15 — Start

16 — Hold

17

fig-14

19   20   21

18   22

EP 0 446 975 B1